# FASCICULE DE BREVET EUROPEEN

(11) **EP 3 506 901 B1**
(45) Date de publication et mention de la délivrance du brevet: **24.03.2021**
(21) Numéro de dépôt: 17780788.0
(22) Date de dépôt: 05.09.2017
(51) Int. Cl.: A61K 31/47, A61P 21/00

(54) **UTILISATION DE L'ACIDE KYNURÉNIQUE DANS LE TRAITEMENT DE L'ATROPHIE MUSCULAIRE**
VERWENDUNG VON KYNURENSÄURE ZUR BEHANDLUNG VON MUSKELATROPHIE
USE OF KYNURENIC ACID FOR TREATING MUSCLE ATROPHY

(30) Priorité: 05.09.2016 FR 1658239
(43) Date de publication de la demande: 10.07.2019
(73) Titulaire: Metabrain Research, 94700 Maisons-Alfort (FR)
(72) Inventeur: RAYNAL, Sophie, N., 75016 Paris (FR); AUDET, Annick, 91630 Leudeville (FR); AUTIER, Valérie, 91190 Gif-sur-Yvette (FR); CHARON, Christine, 91940 Gometz-le-Chatel (FR); DURAND, Jean-Denis, 93100 Montreuil sous bois (FR); KERGOAT, Micheline, 91940 Les Ulis (FR)
(74) Mandataire: Delaveau, Sophie
(86) Numéro de dépôt international: PCT/FR2017/052338
(87) Numéro de publication internationale: WO 2018/042141

(56) Documents cités:
- WO-A2-2006/117624
- WO-A2-2010/007085
- TURSKI M.P. ET AL.: "Kynurenic Acid Content in Selected Culinary Herbs and Spices", JOURNAL OF CHEMISTRY, vol. 2015, 2015, pages 1-6, XP002772471,
- WALKER LANCE S ET AL: "Chromium picolinate effects on body composition and muscular performance in wrestlers", MEDICINE AND SCIENCE IN SPORTS AND EXERCISE, vol. 30, no. 12, décembre 1998 (1998-12), pages 1730-1737, XP002776096, ISSN: 0195-9131
- CAMPBELL WAYNE W ET AL: "Effects of resistance training and chromium picolinate on body composition and skeletal muscle in older men", JOURNAL OF APPLIED PHYSIOLOGY, vol. 86, no. 1, janvier 1999 (1999-01), pages 29-39, XP002776097, ISSN: 8750-7587
- VINCENT JOHN B: "The potential value and toxicity of chromium picolinate as a nutritional supplement, weight loss agent and muscle development agent.", SPORTS MEDICINE (AUCKLAND, N.Z.) 2003, vol. 33, no. 3, 2003, pages 213-230, XP9501921, ISSN: 0112-1642

### SEQUENCE LISTING

<110> Metabrain Research
<120> UTILISATION DE METABOLITES DU TRYPTOPHANE DANS LE TRAITEMENT DE L'ATROPHIE MUSCULAIRE
<130> 1H308550 0005
<160> 6
<170> PatentIn version 3.5
<210> 1
   <211> 22
   <212> DNA
   <213> Artificial Sequence
<220>
   <223> mr myostatin d
<400> 1
   gagtctgact ttctaatgca ag 22
<210> 2
   <211> 20
   <212> DNA
   <213> Artificial Sequence
<220>
   <223> mr myostatin ind
<400> 2
   tgttgtagga gtcttgacgg 20
<210> 3
   <211> 20
   <212> DNA
   <213> Artificial Sequence
<220>
   <223> mh Atrogin d
<400> 3
   agagtcggca agtctgtgct 20
<210> 4
   <211> 19
   <212> DNA
   <213> Artificial Sequence
<220>
   <223> mh Atrogin ind
<400> 4
   gtgaggcctt tgaaggcag 19
<210> 5
   <211> 20
   <212> DNA
   <213> Artificial Sequence
<220>
   <223> m beta-actin d
<400> 5
   ctctagactt cgagcaggag 20
<210> 6
   <211> 19
   <212> DNA
   <213> Artificial Sequence
<220>
   <223> m beta-actin ind
<400> 6
   ggtaccacca gacagcact 19

## Description

La présente invention concerne l'acide kynurénique pour l'utilisation dans le traitement et la prévention des maladies liées à l'atrophie musculaire.

### DOMAINE DE L'INVENTION

Sur le plan fonctionnel, il est important de dissocier la puissance musculaire impliquée dans beaucoup d'activités basiques (transfert assis-debout, montée d'escalier, marche), de la force soutenue (capacité à maintenir un niveau de contraction maximal lors d'un effort soutenu) et de la qualité musculaire (mesure de force par unité de masse musculaire) qui peuvent se dégrader en fonction des situations comme dans certaines maladies associées à une fragilité ou atrophie musculaire (la cachexie, la sarcopénie, l'obésité sarcopénique, le cancer, la myopathie de Duchenne (DMD), la sclérose amyotrophique latérale (SLA), la dystrophie myotonique (MDA), l'insuffisance cardiaque...), ou lors de traumas musculaires consécutifs à un effort physique trop intense. Le coefficient d'endurance isocinétique (rapport de force entre les trois dernières contractions musculaires concentriques par rapport aux trois premières) et l'évolution de la perte de force musculaire sont généralement de bons indicateurs quelle que soit la situation.

Le vieillissement de la fonction musculaire ou le déclin musculaire par la diminution progressive de la masse et de la performance et/ou de la force musculaire, appelée aussi sarcopénie est responsable d'importantes complications telles que l'augmentation du nombre de chutes, une diminution de l'autonomie physique, des altérations du système immunitaire. Les mécanismes impliqués sont multiples et complexes comme la sédentarité, la baisse d'activité physique, un mauvais statut nutritionnel, un état inflammatoire sous-jacent, mais aussi des facteurs hormonaux et neurogéniques, comme un déséquilibre entre la dégradation et la synthèse protéique musculaire qui conduisent *in fine* à une atrophie des fibres musculaires et la réduction de la capacité à produire une force. On retrouve également ces mêmes mécanismes, dont la contribution varie selon la situation, dans le cas d'immobilisation prolongée lors de cancer, d'insuffisance cardiaque ou rénale, ou autres maladies graves chroniques et aiguës (la cachexie, l'obésité sarcopénique, la myopathie de Duchenne (DMD), la sclérose amyotrophique latérale (SLA), la dystrophie myotonique (MDA)) mais aussi consécutivement à un trauma musculaire ou un effort physique trop intense.

Chez les personnes âgées par exemple, alors que les acides aminés sont détournés du muscle, l'insulino-résistance dont la prévalence augmente avec l'âge, joue un rôle défavorable en augmentant la protéolyse des protéines musculaires conduisant à une perte de la masse et de la force musculaires (Bauer et al, 2013; Biolo et al. 2014). De même, la diminution du taux des hormones anaboliques (testostérone, GH-IGF1, DHEA) comme l'augmentation du taux des cytokines pro-inflammatoires (notamment l'IL-6 et le TNF-α) amplifient le processus de protéolyse (Bosutti et al, 2008; Biolo et al, 2008; Guillet et al, 2009). Par ailleurs, la diminution de l'activation des cellules satellites responsables de la régénération musculaire, l'augmentation du taux de myostatine (ou GDF-8) connue pour être exprimée dans le muscle squelettique et jouer un rôle inhibiteur de la croissance et du développement musculaire (Lee, 2010), de l'atrogin-1 (ou MAFbx) et de MURF-1, le vieillissement mitochondrial et l'apoptose (conduisant à la mort cellulaire programmée) contribuent également à ce phénomène qui peut être aussi exacerbé par une augmentation de la graisse intramusculaire dans le cas d'une obésité sarcopénique (Beyer et al. 2012). A l'inverse, des mutations génétiques dans le gène de la myostatine par exemple, augmentent la masse du muscle squelettique chez les animaux, due à une croissance à la fois hyperplasique et hypertrophique des myofibres (McPherron et Lee, 2002 et 2003; Bass et al, 1999).

Alors que le renforcement du muscle peut être au moins en partie compensé par une augmentation d'activité physique et par un apport supplémentaire en protéines alimentaires (Deutz et al, 2014), la prévention ou le traitement de la sarcopénie reposent aujourd'hui uniquement sur un programme d'activité physique régulier adapté à chacun et une surveillance des apports protéino-énergétiques. Les recommandations actuelles préconisent des activités physiques en endurance (aérobie) mais également des activités de renforcement musculaire (contre résistance), ainsi que des exercices sollicitant spécifiquement l'équilibre. D'un point de vue thérapeutique, la testostérone et l'hormone de croissance (GH) n'améliorent les performances musculaires que pour les sujets hypogonadiques ou ayant une déficience en GH ; la DHEA ne montre malheureusement aucun bienfait en termes de performance musculaire ; la vitamine D diminue le risque de chute, sans amélioration directe de la force ou de la puissance musculaire. Ainsi, d'autres axes de recherche sont actuellement menés afin de définir de nouvelles approches thérapeutiques et préventives. Des modulateurs de certains récepteurs aux androgènes sélectifs ou SARMs, ainsi que des inhibiteurs de myostatine actuellement en cours d'étude pourraient s'avérer bénéfiques comme certains acides aminés apportés sous forme de compléments alimentaires notamment chez les personnes âgées malnutries.

Le tryptophane (TRP), est un acide aminé essentiel nécessaire à la biosynthèse des protéines et est également le précurseur de plusieurs molécules biologiques. Métabolisé essentiellement par la voie de la kynurénine (KP), le tryptophane génère de nombreux métabolites (au moins une centaine) comme la kynurénine (KYN), l'acide kynurénique (KA), l'acide anthranilique (AA), l'acide xanthurénique, l'acide quinolinique (QUIN), l'acide picolinique (PICO), l'acide quinaldique (QL-Dic), la 3-OH-kynurénine notamment (Widner B et al, 1997), et constitue également une source importante pour la synthèse de novo de NAD⁺ ou Nicotinamide-Adénine-Dinucléotide.

Il a été montré qu'une supplémentation en TRP chez des souris soumises à un régime pauvre en protéine, est capable de réduire la perte de masse musculaire en augmentant le contenu en IGF-1 dans les muscles et modifiant l'expression de gènes jouant un rôle important dans la synthèse protéique, le développement musculaire ou la taille des fibres (Dukes A. et al, 2015), tandis que l'effet de la L-kynurénine varie selon la dose testée (bénéfique ou négatif). Des études anciennes avaient déjà mis en évidence l'effet bénéfique du TRP sur le muscle, sa morphologie, et la synthèse protéique (Sanfilippo et al, 1995 ; Lin et al, 1988). Toutefois, aucun document ne décrit ni ne suggère que les métabolites du tryptophane choisis parmi l'acide kynurénique, l'acide anthranilique, l'acide quinolinique, l'acide picolinique et l'acide quinaldique puissent avoir un effet positif sur la masse musculaire. Il a même au contraire été prouvé dans l'exemple 1 selon l'invention qu'un des métabolites du tryptophane, la 3OH-Kynurenine, n'avait pas d'effet sur synthèse protéique dans des cellules C2C12.

En l'absence de traitements efficaces à ce jour, il convient de penser que les individus et plus largement les mammifères présentant une baisse de performance musculaire et/ou force/puissance musculaire pourraient largement bénéficier de traitements médicamenteux, d'aliments enrichis ou de compléments alimentaires destinés à maximiser l'anabolisme et diminuer le catabolisme du tissu musculaire.

Alors que la myostatine est un régulateur négatif du développement musculaire chez plusieurs espèces comme l'homme, l'inhibition de l'activité de l'enzyme et de son expression représente de fait une approche ou intervention thérapeutique d'intérêt pour le traitement et la prévention de dysfonctionnements musculaires.

### Description détaillée de l'invention

La présente invention concerne donc l'acide kynurénique (KA), ou un énantiomère, diastéréoisomère, hydrate, solvate, tautomère, mélange racémique ou sel pharmaceutiquement acceptable de celui-ci pour utilisation à titre de médicament destiné à l'augmentation et/ou au maintien de la masse et/ou de la puissance musculaire chez un mammifère.

Le composé utilisé dans le cadre de la présente invention est l'acide kynurénique (KA), qui peut être utilisé seul ou en association avec un ou plusieurs parmi l'acide anthranilique (AA), l'acide quinolinique (QUIN), l'acide picolinique (PICO) et l'acide quinaldique (QL-Dic).

Avantageusement, le ou les métabolites/produits selon l'invention sont destinés au traitement et/ou à la prévention de l'atrophie musculaire chez les mammifères et/ou à la limitation de l'atrophie musculaire chez les mammifères et/ou à favoriser l'accroissement musculaire des mammifères faisant de l'exercice et visant à augmenter la masse et/ou la qualité et/ou la puissance musculaire, en prévention de l'apparition de symptômes sarcopéniques ou en rééducation suite à une perte musculaire et/ou à améliorer le temps de récupération après un effort physique intense.

En particulier, l'atrophie musculaire est liée à l'âge et/ou aux conséquences d'un traitement médicamenteux et/ou à une maladie en rapport avec les anomalies de la dystrophine et/ou à une immobilisation et/ou à la cachexie et/ou à une anorexie nerveuse et/ou à une situation de dénutrition et/ou à des dysphagies conséquentes à des situations pathologiques.

Avantageusement, l'atrophie musculaire est la pré-sarcopénie, la sarcopénie ou la sarcopénie sévère. De façon avantageuse, la pré-sarcopénie, la sarcopénie ou la sarcopénie sévère est liée au vieillissement, à l'obésité ou à des maladies chroniques comme le diabète ou l'insuffisance cardiaque.

Dans un mode de réalisation particulier, l'atrophie musculaire est liée à une maladie en rapport avec les anomalies de la dystrophine, en particulier choisie parmi la myopathie de Duchenne, la myopathie de Becker, la sclérose latérale amyotrophique et la dystrophie myotonique de Steinert.

Dans encore un autre mode de réalisation particulier, l'atrophie musculaire est liée à une immobilisation, en particulier quelle qu'en soit la cause, par exemple due à la faiblesse liée à l'âge, à des traumatismes musculaires ou à une hospitalisation (par exemple, récupération post fracture, accompagnement pré/post chirurgie bariatrique, brûlures), à un accident ou à une opération chirurgicale, comme par exemple la pose d'une prothèse du genou ou de la hanche.

Dans un autre mode de réalisation particulier, l'atrophie musculaire est liée à la cachexie, en particulier la cachexie associée à une maladie chronique choisie parmi le cancer, le syndrome d'immunodéficience acquise (SIDA), la bronchopneumopathie chronique obstructive (BPCO), l'insuffisance cardiaque, l'insuffisance hépatique, la tuberculose, l'insuffisance rénale chronique terminale (IRCT) et les maladies inflammatoires chroniques de l'intestin (MICI).

Dans un autre mode de réalisation particulier, l'atrophie musculaire est liée à des troubles du comportement alimentaire comme l'anorexie nerveuse.

Dans un autre mode de réalisation particulier, l'atrophie musculaire est liée à des dysphagies conséquentes à des situations pathologiques (par exemple, post accident vasculaire cérébral (AVC), maladie de Parkinson, dystrophie musculaire oculo-pharyngée (DMOP)).

En particulier le mammifère à qui on administre le métabolite/produit selon l'invention souffre en outre de maladies métaboliques comme le diabète, l'obésité, la stéatose hépatique non alcoolique (NAFLD) ou stéato-hépatite non alcoolique (NASH), de maladies inflammatoires chroniques de l'intestin (MICI), de cancer, d'insuffisance rénale ou cardiaque, de pathologies neurodégénératives ou de troubles psychiatriques comme la dépression.

En effet dans le cas où le mammifère souffre de ces maladies métaboliques telles que diabète, obésité, stéatose hépatique non alcoolique (NAFLD) ou stéato-hépatite non alcoolique (NASH), maladies inflammatoires chroniques de l'intestin (MICI), cancer, insuffisance rénale ou cardiaque, pathologies neurodégénératives ou troubles psychiatriques comme la dépression, le métabolite selon l'invention va avoir en outre un effet favorable sur cette maladie, en dehors des effets décrits ci-dessus.

Le métabolite/produit selon l'invention est également utile pour favoriser l'accroissement musculaire des mammifères faisant de l'exercice et visant à augmenter la masse et/ou la qualité et/ou la puissance musculaire, en prévention par exemple de l'apparition de symptômes sarcopéniques liés à l'âge ou en rééducation suite à une perte musculaire et/ou pour améliorer le temps de récupération après un effort physique intense.

Le mammifère peut être un animal (animal de compagnie comme le chien ou le chat) ou autre animal (bovins, porcins, ovins, caprins, équidés) ou un être humain, de façon avantageuse il s'agit d'un être humain.

Les inventeurs ont découvert que les métabolites/produits selon la présente invention permettaient d'augmenter la synthèse des protéines dans des cellules musculaires C2C12, de diminuer l'expression du gène de la myostatine dans les cellules musculaires C2C12 et/ou d'augmenter le diamètre des myotubes de cellules C2C12 et donc la taille de ces fibres musculaires.

La présente invention concerne de plus l'utilisation d'un métabolite du tryptophane/produit selon l'invention tel que défini ci-dessus pour la fabrication d'un médicament destiné à l'augmentation et/ou au maintien de la masse et/ou de la puissance musculaire chez un mammifère et/ou au traitement et/ou à la prévention de l'atrophie musculaire chez les mammifères et/ou à la limitation de l'atrophie musculaire chez les mammifères et/ou pour favoriser l'accroissement musculaire des mammifères faisant de l'exercice et visant à augmenter la masse et/ou la qualité et/ou la puissance musculaire, en prévention de l'apparition de symptômes sarcopéniques ou en rééducation suite à une perte musculaire et/ou à améliorer le temps de récupération après un effort physique intense.

Elle concerne enfin une méthode pour maintenir et/ou augmenter la masse et/ou la puissance musculaire chez un mammifère, pour le traitement et/ou le traitement prophylactique et/ou pour retarder l'apparition de l'atrophie musculaire chez les mammifères et/ou pour limiter l'atrophie musculaire chez les mammifères et/ou pour favoriser l'accroissement musculaire des mammifères faisant de l'exercice et visant à augmenter la masse et/ou la qualité et/ou la puissance musculaire, en prévention de l'apparition de symptômes sarcopéniques ou en rééducation suite à une perte musculaire et/ou pour améliorer le temps de récupération après un effort physique intense comprenant l'administration d'une quantité efficace d'un métabolite du tryptophane/produit selon l'invention à un sujet qui en a besoin.

La quantité efficace sera adaptée selon la nature et la sévérité du symptôme à traiter, la voie d'administration et aussi le poids et l'âge du sujet. En général l'unité de doses moyenne variera entre une dose de 50 à 300 mg de métabolite/produit, en particulier d'acide kynurénique (KA), par jour en une prise ou plusieurs, lorsque le sujet est un être humain.

L'invention peut ainsi être avantageusement utilisée dans différentes situations de dénutrition, ou situations associées à une fragilité ou atrophie musculaire : sarcopénie (liée au vieillissement, à l'obésité ou à des maladies chroniques comme le diabète ou l'insuffisance cardiaque), cachexie associée à certaines maladies (comme notamment le cancer, le syndrome d'immunodéficience acquise (SIDA), la bronchopneumopathie chronique obstructive (BPCO), l'insuffisance rénale chronique terminale (IRCT)), dysphagies conséquentes à des situations pathologiques (par exemple, post accident vasculaire cérébral (AVC), maladie de Parkinson, dystrophie musculaire oculo-pharyngée (DMOP), traumatismes musculaires ou hospitalisation (par exemple, récupération post fracture, accompagnement pré/post chirurgie bariatrique, brûlures), anorexie nerveuse, maladies rares (telles que la myopathie de Duchenne (DMD), la sclérose amyotrophique latérale (SLA), la dystrophie myotonique (MD),...). L'invention peut également servir en médecine sportive pour améliorer par exemple le temps de récupération après un effort physique intense, ou rentrer dans la composition de produits vétérinaires pour augmenter la masse et/ou qualité musculaire de certains animaux.

Dans un mode de réalisation avantageux, le métabolite/produit selon l'invention se trouve sous une forme purifiée par exemple obtenue par synthèse chimique ou sous la forme d'un extrait de plante (brut ou partiellement purifié) obtenu par des méthodes bien connues de l'homme du métier (macération, percolation etc..) dans un solvant polaire ou organique ou un mélange de ceux-ci.

En effet, le tryptophane et ses métabolites tels que l'acide kynurénique par exemple sont relativement abondants dans l'alimentation traditionnelle ou certaines herbes comme les tubercules de pommes de terre, les miels, le brocoli, les herbes médicinales (Turski MP et al, 2011; Turski MP et al, 2012; Donarski et al, 2010).

Ainsi, il est possible de fournir la dose bénéfique de métabolites actifs du TRP/produit selon l'invention par la consommation d'un complément alimentaire ou par ingestion d'aliments enrichis (par exemple produit laitier ou boisson) avec un extrait ou des substances actives purifiées naturelles ou synthétiques.

Avantageusement, le métabolite/produit selon l'invention se trouve sous la forme d'une composition pharmaceutique ou vétérinaire comprenant un excipient pharmaceutiquement acceptable. Il peut également se trouver sous la forme d'une composition nutraceutique ou d'un complément alimentaire destiné à une administration par voie orale.

Dans un mode de réalisation avantageux, la composition pharmaceutique, vétérinaire, nutraceutique ou le complément alimentaire selon la présente invention comprend en outre un autre agent actif, ayant avantageusement un effet complémentaire ou synergique.

Ce second agent actif peut être administré dans la même composition pharmaceutique ou nutraceutique ou vétérinaire ou le même complément alimentaire que le métabolite de la présente invention. Il peut être aussi administré séparément, soit au même moment soit de façon étalée dans le temps.

Cet agent actif peut-être un ou plusieurs médicaments ou compléments alimentaires ou aliments ou produits vétérinaires ou anticorps couramment utilisés dans la prévention ou le traitement de dysfonctionnements musculaires ou la réduction de la masse musculaire, ce qui pourrait créer des synergies pharmacologiques utiles avec les métabolites selon l'invention, en fonction de la situation (sarcopénie, obésité sarcopénique, insuffisance cardiaque ou rénale, anorexie, cachexies liées au cancer ou autres maladies chroniques, myopathie de Duchenne (DMD), sclérose amyotrophique latérale (SLA), dystrophie myotonique (MD), dysphagies liées à certaines pathologies, traumatismes musculaires ou hospitalisations, chirurgie bariatrique, effort physique intense...).

Cet agent actif peut correspondre à des produits nutritionnels tels que des mélanges de protéines (telles que créatine) ou d'acides aminés (tels que par exemple la lysine, l'arginine, la leucine, le béta-hydroxy béta methylbutryrate, la citrulline), des vitamines (telle que la vitamine D, les vitamines B,...), des minéraux (tels que le magnésium, le calcium,...), ou d'autres agents nutraceutiques connus pour leurs propriétés anti-inflammatoires (tels que les acides gras poly-insaturés oméga-3 (DHA, EPA)), ou d'autres nutriments actifs facilitant son action cellulaire tels que les phospholipides (par exemple phosphatidylcholine, phosphatidylserine).

Cet agent actif peut également correspondre à certaines hormones (telles que l'hormone de croissance (GH), l'insulin-like growth factor (IGF-1)) pour optimiser leurs effets et potentiellement diminuer leurs effets secondaires.

Cet agent actif peut en outre correspondre à des traitements médicamenteux (tels que des antagonistes des récepteurs de l'angiotensine II, ou des modulateurs de récepteurs androgéniques (SARMs), ou encore des inhibiteurs ou anticorps de la myostatine).

Cet agent actif peut de plus correspondre à des agents chondroprotecteurs (tels que la glucosamine, le sulfate de chondroitine, l'acide hyaluronique, ou des hydrolysats de collagène) dans le but de renforcer la masse musculaire altérée par l'immobilité chez les personnes atteintes d'ostéoarthrite.

### DEFINITIONS

Dans le cadre de la présente invention on entend par «pharmaceutiquement acceptable» ce qui est utile dans la préparation d'une composition pharmaceutique ou vétérinaire qui est généralement sûre, non toxique et ni biologiquement ni autrement non souhaitable et qui est acceptable pour une utilisation vétérinaire de même que pharmaceutique humaine.

Dans le cadre de la présente invention on entend par «sels pharmaceutiquement acceptables d'un métabolite ou produit» des sels qui sont pharmaceutiquement acceptables, comme défini ici, et qui possèdent l'activité pharmacologique souhaitée du métabolite parent. Il s'agit ainsi de sels d'addition organiques et inorganiques de l'acide et des sels d'addition de bases) qui sont physiologiquement tolérés et ne produisent aucune réaction allergique ou fâcheuse similaire, comme des étourdissements, lorsqu'elles sont administrées à un être humain ou un animal. Des exemples de sels comprennent, mais sans s'y limiter: acétate, adipate, alginate, aspartate, benzoate, benzènesulfonate, bisulfate, butyrate, citrate, camphorate, camphorsulfonate, cyclopentylpropionate, le digluconate, le dodécylsulfate, l'éthanesulfonate, le fumarate, flucoheptanoate, glycerophosphate, hémisulfate, heptanoate, l'hexanoate, le chlorhydrate, le bromhydrate, l'iodhydrate, le méthanesulfonate de 2-hydroxyéthyle, le lactate, le maléate, le méthanesulfonate, le 2-naphtalènesulfonate, le nicotinate, l'oxalate, le palmoate, le pectinate, le persulfate, le phénylpropionate, le picrate, le pivalate, le propionate, le succinate, le tartrate, le thiocyanate, le tosylate , undécanoate, et analogues. D'autres exemples de sels comprennent les anions des composés de la présente invention mélangé avec un cation approprié tel que Na +, NH4 + et NW4 + (où W est un groupe alkyle en C1-4) ...

Dans le cadre de la présente invention on entend par «solvate d'un métabolite ou produit», tout composé obtenu par addition d'une molécule de solvant inerte sur le métabolite/produit selon l'invention, le solvate se formant en raison de leur force d'attraction mutuelle. Les solvates sont par exemple des alcoolates du composé. Un hydrate est un solvate dans lequel le solvant inerte utilisé est l'eau. Il peut être mono, di ou trihydraté.

Dans le cadre de la présente invention on entend par «tautomère» tout isomère de constitution des métabolites selon la présente invention qui sont interconvertibles par la réaction chimique réversible appelée tautomérisation. Dans la plupart des cas, la réaction se produit par migration d'un atome d'hydrogène accompagnée d'un changement de localisation d'une double liaison. Dans une solution d'un composé capable de tautomérisation, un équilibre entre les 2 tautomères se crée. Le rapport entre tautomères est alors fonction du solvant, de la température et du pH. La tautomérie est donc la transformation d'un groupement fonctionnel en un autre, le plus souvent par déplacement concomitant d'un atome d'hydrogène et d'une liaison π (liaison double ou triple). Des tautomères courants sont par exemple les paires aldéhydes / cétones - alcools ou plus précisément énol; amides - acides imidiques; lactames - lactimes; imines - énamines; énamines - énamines. En particulier il peut inclure une tautomérie cycle - chaîne qui a lieu lorsque le mouvement du proton est accompagné par la transformation d'une structure ouverte à un cycle. L'expression « excipient » signifie un agent non toxique utilisé dans la formulation de compositions pharmaceutiques, nutraceutiques ou vétérinaires ou de complément alimentaire pour fournir un milieu, et/ou une forme utilisable pour une composition pharmaceutique, nutraceutique ou vétérinaire ou un complément alimentaire. Un véhicule peut comprendre un ou plusieurs de ces agents, tel qu'un stabilisant, ou une solution aqueuse à pH tamponné. Des exemples d'excipient pharmaceutiquement acceptables ou utilisables dans une composition nutraceutique ou un complément alimentaire comprennent des ingrédients tampons aqueuses ou solides, y compris le phosphate, le citrate et d'autres acides organiques; des antioxydants comprenant l'acide ascorbique; polypeptides de bas poids moléculaire (moins d'environ 10 résidus); des protéines telles que la sérumalbumine, la gélatine ou des immunoglobulines; des polymères hydrophiles tels que la polyvinylpyrrolidone; des acides aminés tels que la glycine, la glutamine, l'asparagine, l'arginine ou la lysine; des monosaccharides, des disaccharides et d'autres glucides comprenant le glucose, le mannose ou des dextrines; des agents chélatants tels que l'EDTA; des alcools de sucre tels que le mannitol ou le sorbitol; contre-ions formant sel tels que le sodium; et / ou des agents tensioactifs non ioniques tels que le TWEEN®, le polyéthylèneglycol (PEG), et PLURONICS®.

La composition pharmaceutique, nutraceutique ou vétérinaire ou le complément alimentaire est préparée afin de s'adapter au mode d'administration. Les excipients pharmaceutiques ou nutraceutiques acceptables sont déterminés en partie par la composition administrée, ainsi que par le procédé particulier utilisé pour administrer la composition. En conséquence, il existe une grande variété de formulations appropriées de compositions pharmaceutiques, nutraceutiques ou vétérinaires ou de complément alimentaire qui pourraient contenir les métabolites/produits qui sont décrits ici. Le dosage de ces métabolites/produits à administrer dépend du cas individuel et, comme d'habitude, doit être adapté aux circonstances individuelles pour obtenir une quantité thérapeutique efficace et un effet optimum. Ainsi, il dépend de la nature et du degré de sévérité de l'affection à traiter et également de la progression de la maladie, ainsi que de l'âge, de l'état de santé général du patient et de la réponse individuelle de l'homme ou de l'animal à traiter. La dose quotidienne peut être administrée en une seule dose ou, en particulier lorsque de plus grandes quantités sont administrées, être divisée en plusieurs doses individuelles.

Les compositions peuvent être sous forme solide, liquide ou semi-solide, adaptée aux diverses voies d'administration (orale, rectale, nasale, intra oculaire, locale - par exemple, topique, transdermique, buccale, vaginale ou sublinguale), parentérale (par exemple sous-cutanée, intramusculaire, intraveineuse ou intradermique). L'administration orale est préférée, cette voie est la plus appropriée pour des traitements chroniques. Cependant, l'administration par d'autres voies est possible, par exemple par voie intraveineuse et transdermique. Les formulations intraveineuses contiennent la substance active dissoute, en suspension ou émulsionnée dans un véhicule stérile, éventuellement en présence d'agents émulsifiants, des stabilisants, des agents tampons et d'autres additifs classiques; elles sont normalement réparties dans des fioles ou des flacons pour perfusion, et peuvent être stockées sous forme de produits secs à reconstituer avec de l'eau ou avec un véhicule approprié avant utilisation. Les compositions pharmaceutiques solides peuvent être des comprimés, des gélules, des poudres, des granules, des pilules, des poudres à reconstituer etc... ; elles peuvent contenir des excipients classiques tels que des liants, des charges, des diluants, des agents de compression, des lubrifiants, des détergents, des colorants, des agents aromatisants et des agents mouillants. Les comprimés peuvent être enrobés conformément à des procédés bien connus dans le domaine technique. Des charges appropriées comprennent la cellulose, le mannitol, le lactose et d'autres agents similaires. Les compositions liquides pour administration orale peuvent être sous forme de suspensions aqueuses ou huileuses, de solutions, d'émulsions, de sirops ou d'élixirs ou peuvent être présentées sous forme de produits secs pour reconstitution avec de l'eau ou un véhicule approprié avant utilisation; Elles peuvent contenir des additifs classiques, par exemple des agents de suspension tels que le sorbitol, le sirop, la méthylcellulose, la gélatine, l'hydroxyéthylcellulose, la carboxyméthylcellulose, un gel de stéarate d'aluminium ou des matières grasses comestibles hydrogénées, des émulsifiants tels que la lécithine, le monooléate de sorbitane ou la gomme arabique; des transporteurs non aqueux (qui peuvent comprendre des huiles comestibles) tels que l'huile d'amande, l'huile de noix de coco fractionnée, des esters huileux comme des esters de glycérine, de propylène glycol ou d'alcool éthylique; des conservateurs tels que méthyle ou de propyle p-hydroxybenzoate ou l'acide sorbique et, si cela est désiré, des agents aromatisants ou colorants classiques.

Le terme « prévention » fait référence à une réduction du risque de contracter ou de développer une maladie ou un trouble (par exemple, faire en sorte qu'au moins l'un des symptômes cliniques de la maladie ne puisse se développer) chez un sujet qui peut être exposé à un agent pathogène, ou prédisposé à la maladie avant son apparition.

Le terme « traitement » d'une maladie ou d'un trouble désigne, dans un mode de réalisation, l'amélioration de la maladie ou du trouble (par exemple, en arrêtant la maladie ou la réduction de la manifestation, l'étendue ou la gravité d'au moins un des symptômes cliniques de celui-ci).

Les molécules naturelles ou synthétiques peuvent être utilisées en combinaison avec un support pharmaceutique approprié. De telles compositions comprennent une quantité efficace des métabolites du TRP ou produit selon l'invention, et un support pharmaceutique acceptable ou un excipient.

L'invention sera mieux comprise à la lecture de la description des figures et des exemples qui suivent qui sont donnés à titre indicatif, non limitatif.
La Figure 1 représente l'effet de l'acide kynurénique (KA) sur la synthèse protéique dans des cellules musculaires C2C12 en fonction de la dose utilisée.
La Figure 2 représente l'effet de l'acide anthranilique sur la synthèse protéique dans des cellules musculaires C2C12.
La Figure 3 représente l'effet de l'acide quinolinique sur la synthèse protéique dans des cellules musculaires C2C12.
La Figure 4 représente l'effet de l'acide picolinique sur la synthèse protéique dans des cellules musculaires C2C12.
La Figure 5 représente l'effet de la 3-OH kynurénine sur la synthèse protéique dans des cellules musculaires C2C12.
La Figure 6 représente l'effet de l'acide kynurénique sur l'expression du gène de la myostatine dans des cellules musculaires C2C12.
La Figure 7 représente l'effet de l'acide kynurénique sur les paramètres morphométriques des fibres musculaires C2C12 (Figure 7A-B)
La Figure 8 représente l'effet de l'acide kynurénique sur l'atrophie musculaire induite par une immobilisation de 7 jours chez la souris normale.

### Exemple 1 : mesure de la synthèse protéique dans des cellules C2C12

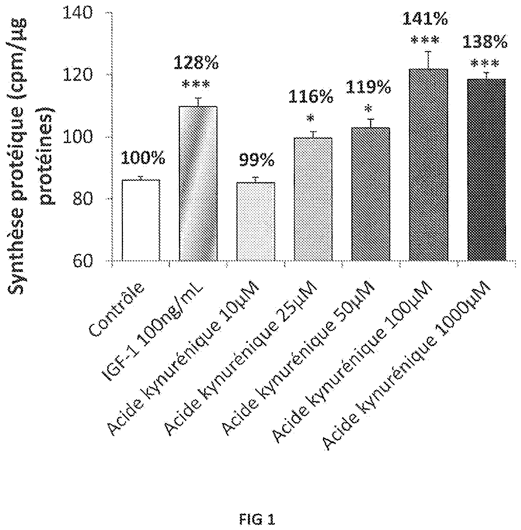

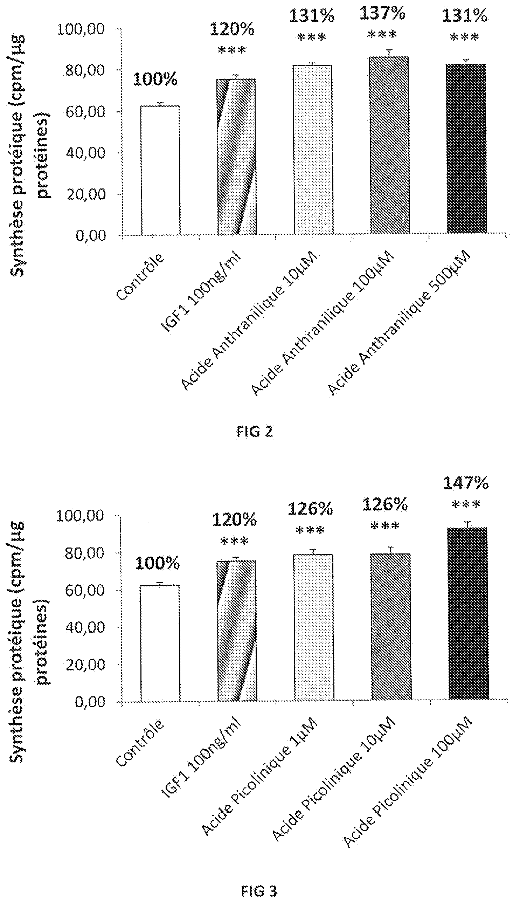

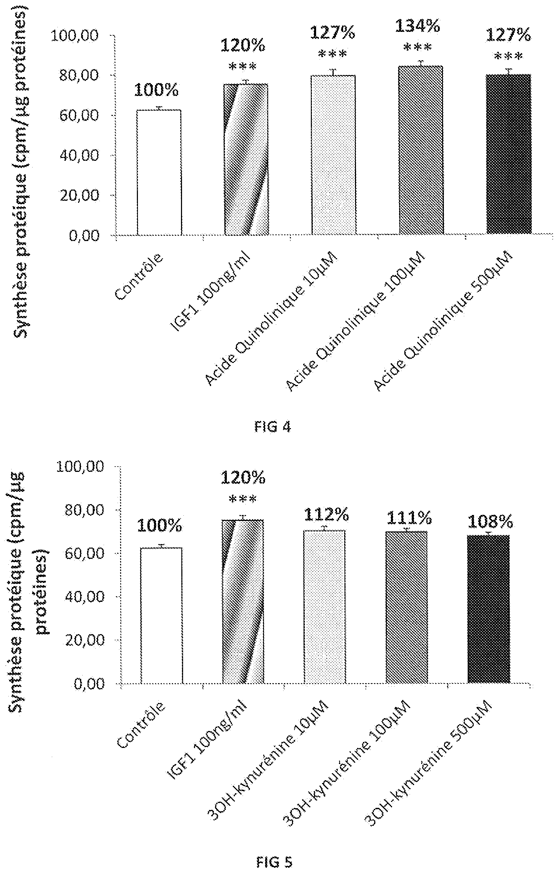

Les cellules sont comptées et ensemencées à la densité de 20000 cellules par puits en plaque 24 puits dans un milieu DMEM contenant du glucose à raison de 4.5 g/L et supplémenté en sérum de veau fœtal (10 %) et en antibiotiques (pénicilline et streptomycine). Quarante-huit heures plus tard, les myoblastes sont induits en différenciation par privation partielle en sérum (2 % au lieu de 10 %) pendant 5 jours. Les cellules sont ensuite placées dans un milieu sans glucose ni leucine (milieu Krebs) pendant 1h à 37°C puis incubées 150 min en présence des produits à tester (DMSO (contrôle), Acide Kynurénique ou Acide Anthralinique ou Acide Quinolinique ou Acide Picolinique ou 3OH-Kynurenine) ou référence (IGF-1, 100 ng/mL) dans un milieu DMEM sans sérum contenant de la leucine radio marquée 2,5 µCi/mL. A la fin de l'incubation, les surnageants sont éliminés et les cellules sont lysées dans une solution de NaOH 0,1N pendant 30 min. La radioactivité est mesurée dans la fraction cellulaire et la quantité de protéines totale est déterminée par dosage selon la méthode Lowry. Chaque condition est évaluée au minimum en n = 6 ; l'IGF-1, 100 ng/mL est notre témoin pour stimuler la synthèse protéique. Les résultats sont exprimés en cpm/µg de protéines après 150min d'incubation ou en pourcentage par rapport à la condition contrôle. Les résultats sont exprimés en % du contrôle et un test statistique est réalisé : test de Dunnett ou Dun (*p<0.05, **p<0.01, ***p<0.001 versus contrôle). Les résultats obtenus avec l'acide kynurénique, l'acide anthranilique, l'acide quinolinique, l'acide picolinique et la 3-OH kynurénine sont représentés sur les figures 1 à 5. Ils montrent que l'acide Kynurénique, l'Acide Anthranilique, l'Acide Quinolinique et l'Acide Picolinique induisent de façon significative la synthèse protéique. Une stimulation dose-dépendante de la synthèse protéique est observée après 150 min d'incubation en présence d'acide kynurénique. En revanche, la 3OH-Kynurénine, un autre métabolite du tryptophane, n'a pas d'effet sur la synthèse protéique.

### Exemple 2 : Mesure de l'expression génique de la myostatine dans des cellules C2C12

Les cellules myoblastiques C2C12 (ATCC CRL-1772) sont ensemencées en plaques 24 puits à la densité de 30 000 cellules par puits et cultivées dans un milieu

DMEM contenant du glucose à raison de 4,5 g/L et supplémenté en sérum de veau fœtal (10%) et en antibiotiques (pénicilline et streptomycine). Quarante-huit heures plus tard, les myoblastes sont induits en différenciation par privation partielle en sérum (2% au lieu de 10%) pendant 5 jours. Les cellules sont ensuite placées dans un milieu appauvri en glucose (DMEM contenant 1 g/L de glucose) et dépourvu de sérum en présence des molécules à tester (DMSO (contrôle) ou IGF-1 (100ng/mL) ou Acide Kynurénique) ou des références (IGF-1 à la concentration de 100 ng/mL) pendant 6 h. A la fin de l'expérience, les ARN messagers (ARNm) sont extraits en utilisant la méthodologie classique à base de phénol et chloroforme. Brièvement, les cellules sont lysées dans une solution de trizol (Sigma T9424) contenant un acide fort et du phénol. Les ARNm sont séparés des protéines par ajout de chloroforme puis centrifugation. Ils sont ensuite précipités dans l'isopropanol pour ensuite être suspendus à la concentration de 1µg/µL dans une eau ultra pure débarrassée des RNases et DNAses. 1µg d'ARNm est alors converti par rétro transcription en ADN complémentaire par l'enzyme AMV en présence d'une amorce et d'un mélange de nucléotides selon le protocole donné par le fournisseur (Applied Biosystems 4368814). L'expression génique est étudiée par réaction en chaine initiée par une enzyme polymérase et couramment nommé PCR dans des conditions quantitatives, d'où le nom précis de qPCR. Les qPCR sont réalisées sur un 7900HT Fast real-Time PCR detection system (Applied Biosystems). Les conditions de programmation sont standard et consistent en 1 cycle à 95°C pendant 15 min, suivi de 40 cycles à 95°C pendant 15 s et à 60 °C pendant 1 min et on termine par une étape de courbe de fusion entre 60°C et 95°C. Les échantillons analysés contiennent tous 100 ng d'ADNc, un tampon de qPCR incluant l'enzyme, le mélange d'oligonucléotides et l'intercalant (le sybergreen), et le couple d'amorces spécifiques du gène étudié, stratégiquement choisi entre deux séquences exoniques et à la concentration finale de 200 nM. Des sondes fluorescentes se fixent sur l'ADN double brin et ne fluorescent qu'une fois fixées à l'ADN. Un seuil de fluorescence est établi par le programme de la machine. Lorsque la quantité d'ADN permet à la sonde fluorescente de dépasser ce seuil, on obtient un numéro de cycle PCR appelé "Ct" pour "Cycle Threshold" ou cycle seuil. C'est cette valeur qui est à la base des calculs pour quantifier l'ADN de façon relative. On établit un rapport R entre la quantité d'ADN de départ d'un échantillon et celle d'un témoin, qui n'a pas subi de traitement (soit R=2^{-(Ct échantillon - Ct témoin)}) et cette mesure qui est rapportée à celle d'un gène ménage connu pour ne pas être modulé par le traitement (soit R = 2^{-ΔΔCt}).

Les amorces utilisées sont consignées dans le tableau 1suivant :
Tableau 1: Amorces utilisées pour évaluer les modifications d'expression
génique

| **Gene** | **Sequence 5' → 3'** | **Nb of bases** | **Tm** | **Accession number** |
|---|---|---|---|---|
| mr myostatin d | GAGTCTGACTTTCTAATGCAAG | 21 | 62 | m: NM_010834 |
| mr myostatin ind | TGTTGTAGGAGTCTTGACGG | 20 | 60 | r: AF019624 |
| mh Atrogin d | AGAGTCGGCAAGTCTGTGCT | 20 | 62 | m: AF441120 |
| mh Atrogin ind | GTGAGGCCTTTGAAGGCAG | 19 | 60 | h: NM_058229 |
| m beta-actin d | CTCTAGACTTCGAGCAGGAG | 20 | 62 | m:X03672 |
| m beta-actin ind | GGTACCACCAGACAGCACT | 19 | 60 | |

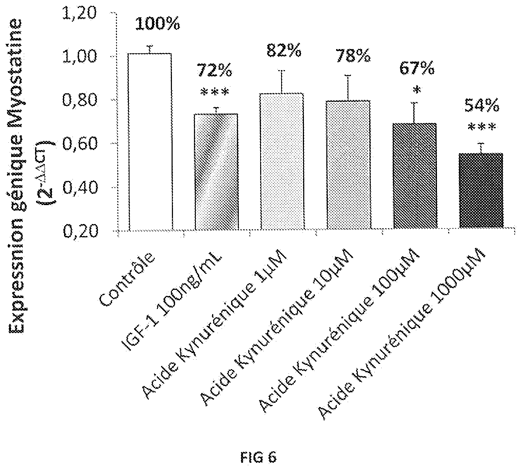

A la fin de l'incubation, l'expression du gène de la myostatine est mesurée par RT-QPCR et normalisée à l'aide du gène ménage de la beta actine. Un test statistique est réalisé : test de Dunnett ou Dun (*p<0.05, **p<0.01, ***p<0.001 versus contrôle). Les résultats obtenus avec l'acide kynurénique sont représentés sur la figure 6. Une inhibition significative dose-dépendante de l'expression du gène de la myostatine est observée après 6 heures d'incubation avec l'acide Kynurénique.

### Exemple 3 : Evaluation du diamètre des fibres musculaires C2C12

Les cellules myoblastiques C2C12 (ATCC CRL-1772) sont ensemencées en plaques 8 puits traitées au glycérol, à la densité de 10 000 cellules par puits et cultivées dans un milieu DMEM contenant du glucose à raison de 4,5 g/L et supplémenté en sérum de veau fœtal (10%) et en antibiotiques (pénicilline et streptomycine). Quarante-huit heures plus tard, les myoblastes sont induits en différenciation par privation partielle en sérum (2 % au lieu de 10 %) pendant 3 jours. Les cellules sont ensuite placées dans un milieu appauvri en glucose (DMEM contenant 1 g/L de glucose) et dépourvu de sérum en présence des molécules à tester (DMSO (contrôle) ou Acide Kynurénique) ou des références (IGF-1 à la concentration de 10 ng/mL ou dexamethasone 10 µM) pendant 3 jours. A la fin de la culture, les cellules sont rincées et fixées à l'aide d'une solution Glutaraldehyde 2,5% / Triton 0,1% pendant 1h à température ambiante. Le tapis cellulaire est recouvert de DAPI (marquage fluorescent du noyau cellulaire). Après stockage à l'obscurité pendant 16h au froid, les lames sont observées sous microscope à fluorescence (Carl Zeiss, AxioVert 200) et les images analysées à l'aide du logiciel Axiovision 4.1 pour mesurer le diamètre des fibres.

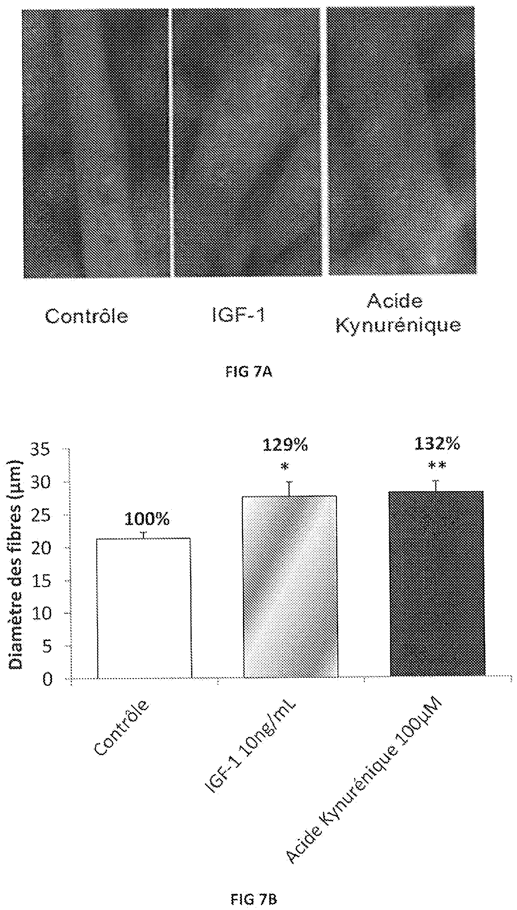

Une image représentative de chaque condition est illustrée. Un test statistique est réalisé : test de Dunnett ou Dun (*p<0.05, **p<0.01, ***p<0.001 versus contrôle). Les résultats obtenus avec l'acide kynurénique sont représentés sur la figure 7. Après incubation pendant 3 jours en présence d'Acide Kynurénique (100µM), une augmentation de la taille des fibres musculaires C2C12 est observée.

### Exemple 4 : effet de l'acide kynurénique sur l'atrophie musculaire induite par l'immobilisation chez la souris

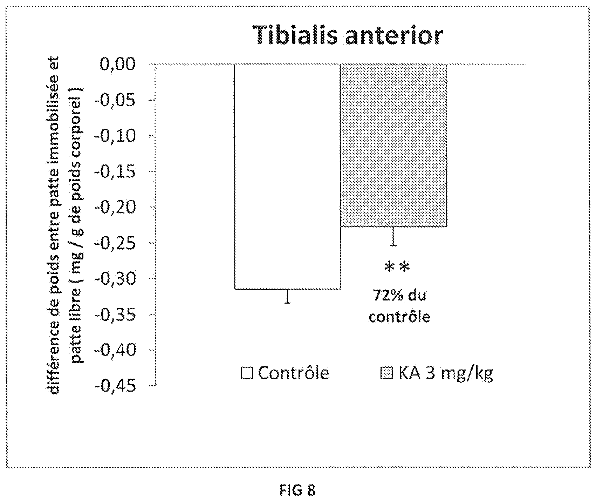

L'immobilisation des membres est connue pour induire une atrophie des muscles squelettiques impliquant des mécanismes liés à la dégradation des protéines, à un changement des types de fibres musculaires, à un stress oxydatif ou des mécanismes inflammatoires. Brièvement, dans cette étude des souris CD1 mâles de 8-9 semaines d'âge ont été utilisées. Le pied de la patte postérieure gauche a été immobilisé durant 7 jours selon la procédure décrite par Caron et al. (2009), en position de flexion dorso-tibiale, au moyen d'une agrafe chirurgicale. L'autre pied (patte postérieure droite) est utilisé comme contrôle. Pendant la durée de l'immobilisation, un groupe d'animaux (n=12) a reçu dans l'eau de boisson un traitement par de l'acide kynurénique (3 mg/kg par jour). Un autre groupe d'animaux a servi de contrôle (n=16 animaux). A l'issue des 7 jours d'étude, les animaux ont été sacrifiés pour prélever le muscle Tibialis anterior sur les deux pattes postérieures. Pour chaque animal, la différence entre le poids du muscle de la patte immobilisée et la patte libre est calculée, puis rapportée au poids corporel. Les résultats sont exprimés en perte de poids de la patte immobilisée par rapport à la patte contrôle (en mg par g de poids corporel), et un test statistique est réalisé: test de Student (*p<0.05, **p<0.01, ***p<0.001 versus groupe contrôle). Les résultats obtenus montrent que l'acide kynurénique diminue de façon significative la perte de masse musculaire (Tibialis anterior) induite par l'immobilisation par rapport au groupe contrôle sans traitement (Figure 8).

### BIBLIOGRAPHIE

Bass J et al., Domest Anim Endocrinol, 1999, 17(2-3): 191-197
Bauer J et al., J Am Med Dir Assoc, 2013, 14: 542-559
Beyer et al., Curr. Opin. Clin. Nutr. Metab. Care, 2012, 15: 12-22
Biolo G et al., Am J Clin Nutr, 2008, 88: 950-958
Biolo et al., Clinical Nutrition, 2014, 33: 737-748
Bosutti A et al., J Clin Endocrinol Metab, 2008, 93: 3226-3229
Caron AZ et al. J Appl Physiol. 2009; 106:2049-2059.
Deutz NE et al., Clin Nutr, 2014, 33(6):929-36.
Donarski J.A. et al. Food Chemistry, 2010, 118: 987-994
Dukes A. et al., Nutrition, 2015, 31 : 1018-1024
Guillet C et al., Obes Rev, 2012, 13(Suppl.2): 51-57
Guillet C et al., J Clin Endocrinol Metab, 2009, 94: 3044-3050
Lee SJ. Immunol Endocr Metab Agents Med Chem., 2010, 10: 183-194
Lin FD, et al., J Nutr., 1988, 118 : 445-449
McPherron AC et al., J Clin Invest. 2002, 109: 595-601
McPherron AC and Lee, Proc. Natl. Acad. Sci. USA 2003, 100 (26): 15842-15846
Sanfilippo et al., Ital J Anat Embryol, 1995, 100: 131-141
Turski M.P., et al. Planta Med., 2011, 77: 858-864
Turski M.P. et al. Plant Foods Hum Nutr, 2012, 67: 17-23
Widner B et al., Clinical Chemistry, 1997, 43: 2424-26

## Revendications

1. Acide kynurénique, ou un énantiomère, diastéréoisomère, hydrate, solvate, tautomère, mélange racémique ou sel pharmaceutiquement acceptable de celui-ci, pour utilisation à titre de médicament destiné à l'augmentation et/ou au maintien de la masse et/ou la puissance musculaire chez un mammifère.

2. Acide kynurénique pour utilisation selon la revendication 1, **caractérisé en ce que** le médicament est destiné au traitement et/ou à la prévention de l'atrophie musculaire chez les mammifères et/ou à la limitation de l'atrophie musculaire chez les mammifères et/ou à favoriser l'accroissement musculaire des mammifères faisant de l'exercice et visant à augmenter la masse et/ou la qualité et/ou la puissance musculaire, en prévention de l'apparition de symptômes sarcopéniques ou en rééducation suite à une perte musculaire et/ou à améliorer le temps de récupération après un effort physique intense.

3. Acide kynurénique pour utilisation selon la revendication 2, **caractérisé en ce que** l'atrophie musculaire est liée à l'âge et/ou aux conséquences d'un traitement médicamenteux et/ou à une maladie en rapport avec les anomalies de la dystrophine et/ou à une immobilisation et/ou à la cachexie et/ou à une anorexie nerveuse et/ou à une situation de dénutrition et/ou à des dysphagies conséquentes à des situations pathologiques.

4. Acide kynurénique pour utilisation selon l'une quelconque des revendications 2 ou 3, **caractérisé en ce que** l'atrophie musculaire est la pré-sarcopénie, la sarcopénie ou la sarcopénie sévère.

5. Acide kynurénique pour utilisation selon la revendication 4, **caractérisé en ce que** la pré-sarcopénie, la sarcopénie ou la sarcopénie sévère est liée au vieillissement, à l'obésité ou à des maladies chroniques comme le diabète ou l'insuffisance cardiaque.

6. Acide kynurénique pour utilisation selon la revendication 3, **caractérisé en ce que** la maladie en rapport avec les anomalies de la dystrophine est choisie parmi la myopathie de Duchenne, la myopathie de Becker, la sclérose latérale amyotrophique et la dystrophie myotonique de Steinert.

7. Acide kynurénique pour utilisation selon la revendication 3, **caractérisé en ce que** la cachexie est associée à une maladie choisie parmi le cancer, le syndrome d'immunodéficience acquise (SIDA), la bronchopneumopathie chronique obstructive (BPCO), l'insuffisance cardiaque, l'insuffisance hépatique, la tuberculose, l'insuffisance rénale chronique terminale (IRCT) et les maladies inflammatoires chroniques de l'intestin (MICI).

8. Acide kynurénique pour utilisation selon l'une quelconque des revendications 1 à 7, **caractérisé en ce que** le mammifère souffre en outre de maladies métaboliques comme le diabète, l'obésité, la stéatose hépatique non alcoolique (NAFLD) ou stéato-hépatite non alcoolique (NASH), de maladies inflammatoires chroniques de l'intestin (MICI), de cancer, d'insuffisance rénale ou cardiaque, de pathologies neurodégénératives ou de troubles psychiatriques comme la dépression, en particulier du diabète.

9. Acide kynurénique pour utilisation selon l'une quelconque des revendications 1 à 8, **caractérisé en ce que** le mammifère est un être humain.

10. Acide kynurénique pour utilisation selon l'une quelconque des revendications 1 à 9, **caractérisé en ce qu'**il se trouve sous une forme purifiée ou sous la forme d'un extrait de plante.

11. Acide kynurénique pour utilisation selon l'une quelconque des revendications 1 à 10, **caractérisé en ce qu'**il se trouve sous la forme d'une composition pharmaceutique ou vétérinaire comprenant un excipient pharmaceutiquement acceptable.

12. Acide kynurénique pour utilisation selon l'une quelconque des revendications 1 à 11, **caractérisé en ce qu'**il se trouve sous la forme d'une composition nutraceutique ou d'un complément alimentaire destiné à une administration par voie orale.

## Patentansprüche

1. Kynureninsäure, oder ein pharmazeutisch unbedenkliches Enantiomer, Diastereomer, Hydrat, Solvat, Tautomer, Racemat oder Salz derselben, zur Verwendung als Arzneimittel, das dazu bestimmt ist, zur Erhöhung und/oder zur Erhaltung der Muskelmasse und/oder - leistungsfähigkeit bei einem Säugetier verwendet zu werden.

2. Kynureninsäure zur Verwendung nach Anspruch 1, **dadurch gekennzeichnet, dass** das Arzneimittel zur Behandlung und/oder zur Verhütung von Muskelatrophie bei Säugetieren und/oder zur Beschränkung von Muskelatrophie bei Säugetieren und/oder dazu bestimmt ist, den Muskelaufbau bei Säugetieren zu fördern, welche sich körperlich betätigen, wobei es weiterhin dazu dient, als vorbeugende Maßnahme, sobald Symptome von Sarkopenie auftreten, oder als rehabilitierende Maßnahme infolge eines Muskelschwunds, die Masse und/oder die Qualität und/oder die Leistungsfähigkeit der Muskeln zu erhöhen, und/oder dazu, nach einer intensiven körperlichen Anstrengung die Erholungsdauer zu verbessern.

3. Kynureninsäure zur Verwendung nach Anspruch 2, **dadurch gekennzeichnet, dass** die Muskelatrophie altersbedingt ist und/oder infolge einer medikamentösen Behandlung auftritt und/oder mit einer Erkrankung einhergeht, welche mit Anomalien hinsichtlich Dystrophin und/oder mit einer Immobilisierung und/oder mit krankhafter Abmagerung und/oder mit einer Magersucht und/oder mit einer Nahrungsmangelsituation und/oder mit Dysphagien infolge pathologischer Situationen in Zusammenhang steht.

4. Kynureninsäure zur Verwendung nach einem beliebigen der Ansprüche 2 oder 3, **dadurch gekennzeichnet, dass** es sich bei der Muskelatrophie um eine Vorstufe von Sarkopenie, um Sarkopenie oder um schwere Sarkopenie handelt.

5. Kynureninsäure zur Verwendung nach Anspruch 4, **dadurch gekennzeichnet, dass** die Vorstufe von Sarkopenie, die Sarkopenie oder die schwere Sarkopenie altersbedingt ist, mit Fettleibigkeit oder mit chronischen Krankheiten wie Diabetes oder Herzinsuffizienz in Verbindung steht.

6. Kynureninsäure zur Verwendung nach Anspruch 3, **dadurch gekennzeichnet, dass** die Erkrankung, welche mit Anomalien hinsichtlich Dystrophin in Zusammenhang steht, aus der Muskeldystrophie Duchenne, der Muskeldystrophie Becker-Kiener, der amyotrophen Lateralsklerose und der myotonen Dystrophie Typ 1 ausgewählt ist.

7. Kynureninsäure zur Verwendung nach Anspruch 3, **dadurch gekennzeichnet, dass** die krankhafte Abmagerung mit einer Erkrankung in Verbindung steht, welche aus Krebserkrankungen, dem erworbenen Immunschwächesyndrom (AIDS), der chronisch-obstruktiven Lungenerkrankung (COPD), einer Herzinsuffizienz, einer Leberinsuffizienz, der Tuberkulose, der chronischen Niereninsuffizienz im Endstadium (ESRD) und den chronisch-entzündlichen Darmerkrankungen (CED) ausgewählt ist.

8. Kynureninsäure zur Verwendung nach einem beliebigen der Ansprüche 1 bis 7, **dadurch gekennzeichnet, dass** das Säugetier darüber hinaus unter Stoffwechselerkrankungen wie Diabetes, Fettleibigkeit, nicht-alkoholbedingter Fettleber (NAFLD) oder nicht-alkoholbedingter Fettleberhepatitis (NASH), chronisch-entzündlichen Darmerkrankungen (CED), Krebserkrankungen, Nieren- oder Herzinsuffizienz, neurodegenerativen Erkrankungen oder psychiatrischen Störungen wie Depressionen leidet, insbesondere unter Diabetes.

9. Kynureninsäure zur Verwendung nach einem beliebigen der Ansprüche 1 bis 8, **dadurch gekennzeichnet, dass** es sich bei dem Säugetier um einen Menschen handelt.

10. Kynureninsäure zur Verwendung nach einem beliebigen der Ansprüche 1 bis 9, **dadurch gekennzeichnet, dass** sie in einer aufgereinigten Form oder in Form eines Pflanzenextrakts vorliegt.

11. Kynureninsäure zur Verwendung nach einem beliebigen der Ansprüche 1 bis 10, **dadurch gekennzeichnet, dass** sie in Form einer pharmazeutischen oder veterinärmedizinischen Zusammensetzung vorliegt, die einen pharmazeutisch unbedenklichen Hilfsstoff umfasst.

12. Kynureninsäure zur Verwendung nach einem beliebigen der Ansprüche 1 bis 11, **dadurch gekennzeichnet, dass** sie in Form einer nutrazeutischen Zusammensetzung oder eines Nahrungsergänzungsmittels vorliegt, welches für eine Verabreichung auf oralem Wege bestimmt ist.

## Claims

1. Kynurenic acid, or an enantiomer, diastereoisomer, hydrate, solvate, tautomer, racemic mixture or pharmaceutically acceptable salt thereof, for use as a medicament intended for increasing and/or maintaining muscle mass and/or power in a mammal.

2. Kynurenic acid for use according to Claim 1, **characterized in that** the medicament is intended for treating and/or preventing muscle atrophy in mammals and/or for limiting muscle atrophy in mammals and/or for promoting muscle growth in mammals performing exercise and aiming to increase muscle mass and/or quality and/or power, for preventing the appearance of symptoms of sarcopenia or for rehabilitation following muscle loss and/or for improving recovery time after an intense physical effort.

3. Kynurenic acid for use according to Claim 2, **characterized in that** the muscle atrophy is related to age and/or to the consequences of a drug treatment and/or to a disease in connection with dystrophin abnormalities and/or to immobilization and/or to cachexia and/or to anorexia nervosa and/or to a situation of undernourishment and/or to dysphagia following pathological situations.

4. Kynurenic acid for use according to either one of Claims 2 and 3, **characterized in that** the muscle atrophy is pre-sarcopenia, sarcopenia or severe sarcopenia.

5. Kynurenic acid for use according to Claim 4, **characterized in that** the pre-sarcopenia, sarcopenia or severe sarcopenia is related to aging, to obesity or to chronic diseases such as diabetes or heart failure.

6. Kynurenic acid for use according to Claim 3, **characterized in that** the disease in connection with dystrophin abnormalities is chosen from Duchenne muscular dystrophy, Becker muscular dystrophy, amyotrophic lateral sclerosis and Steinert's myotonic dystrophy.

7. Kynurenic acid for use according to Claim 3, **characterized in that** the cachexia is associated with a disease chosen from cancer, acquired immunodeficiency syndrome (AIDS), chronic obstructive pulmonary disease (COPD), heart failure, liver failure, tuberculosis, end-stage chronic renal disease (ESRD) and chronic inflammatory bowel diseases (IBDs).

8. Kynurenic acid for use according to any one of Claims 1 to 7, **characterized in that** the mammal is also suffering from metabolic diseases such as diabetes, obesity, non-alcoholic fatty liver disease (NAFLD) or non-alcoholic steatohepatitis (NASH), from chronic inflammatory bowel diseases (IBDs), from cancer, from renal or heart failure, from neurodegenerative pathological conditions or from psychiatric disorders such as depression, in particular from diabetes.

9. Kynurenic acid for use according to any one of Claims 1 to 8, **characterized in that** the mammal is a human being.

10. Kynurenic acid for use according to any one of Claims 1 to 9, **characterized in that** it is in a purified form or in the form of a plant extract.

11. Kynurenic acid for use according to any one of Claims 1 to 10, **characterized in that** it is in the form of a pharmaceutical or veterinary composition comprising a pharmaceutically acceptable excipient.

12. Kynurenic acid for use according to any one of Claims 1 to 11, **characterized in that** it is in the form of a nutraceutical composition or of a food supplement intended for oral administration.
